# EUROPÄISCHE PATENTSCHRIFT

(11) **EP 0 346 953 B1**
(45) Veröffentlichungstag und Bekanntmachung des Hinweises auf die Patenterteilung: **20.01.1993**
(21) Anmeldenummer: 89116154.9
(22) Anmeldetag: 31.10.1985
(51) Int. Cl.: A61K 31/565, A61K 9/14

(54) **4-Hydroxy- bzw. 4-C2-C12-Acyloxy-4-androsten-3,17-dione in mikronisierter Form**
Micronized forms of 4-hydroxy and 4-C2-C12-acyloxy-4-androsten-3,17 diones
Formes micronisées 4-hydroxy et 4-C2-C12-acyloxy-4-androstène-3,17-dione

(30) Priorität: 06.11.1984 CH 5305/84
(43) Veröffentlichungstag der Anmeldung: 20.12.1989
(62) Teilanmeldung aus: 85810500.0
(73) Patentinhaber: CIBA-GEIGY AG, 4002 Basel (CH)
(72) Erfinder: Geller, Leo, Dr., CH-4125 Riehen (CH); Glanzmann, Peter, CH-4053 Basel (CH)

(56) Entgegenhaltungen:
- DE-A- 3 339 295
- US-A- 4 391 755

## Beschreibung

Die Erfindung betrifft 4-Hydroxy- bzw. 4-Acyloxy-4-androsten-3,17-dione der allgemeinen Formel I 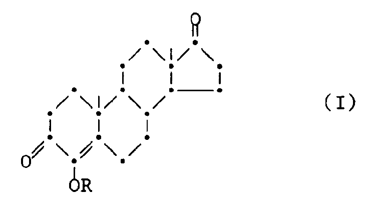
worin R entweder ein Wasserstoffatom oder eine 2 bis 12 Kohlenstoffatome enthaltende gesättigte aliphatische oder aromatische Acylgruppe darstellt, in mikronisierter Form in einer korngröße der Grössenordnung von 2-20 µm.

Als Acylgruppe kommt insbesondere die in der Steroidchemie üblicherweise verwendete Acetyl-, Heptanoyl- oder Benzoylgruppe in Frage.

Die Verbindungen in nicht-mikronisierter Form sind bereits bekannt. Sie werden beschrieben als Aromatasehemmer, die die Umwandlung von 4-Androsten-3,17-dion zu Oestrogenen an menschlichen Placentamikrosomen hemmen (Endocrinology 100 [1977] 1684-1695 und US-A-4,235,893). Die Verbindungen können die Entwicklung von östrogenabhängigen Brusttumoren bei Ratten unterdrücken.

Ferner wird die Verwendung der Verbindungen der allgemeinen Formel I in der DE-A-3 339 295 zur Prophylaxe und Therapie der Prostata-Hyperplasie beschrieben.

Die Verbindungen der allgemeinen Formel I in mikronisierter Form waren jedoch bisher nicht bekannt. Ueberraschenderweise wurde festgestellt, dass die Verbindungen derallgemeinen Formel I besonders geeignet sind für die Herstellung von Trockensubstanzen zur Herstellung von stabilen Suspensionen und von stabilen Suspensionen an sich, die die 4-Hydroxy- oder 4-C₂-C₁₂-Acyloxy-4-androsten-3,17-dione als Wirkstoffe enthalten.

Bevorzugter Gegenstand der Erfindung ist demnach eine Verbindung der allgemeinen Formel I in mikronisierter Form in einer mittleren Korngrösse von 3-6 µm.

Die Verbindungen der allgemeinen Formel I in mikronisierter Form werden hergestellt, indem man eine Verbindung der allgemeinen Formel I in an sich bekannter Weise mikronisiert. Das Mikronisieren des Wirkstoffes geschieht mittels eines Ultraschalldesintegrators (z.B. Branson Sonifier) nach bekannten Verfahren [J. Pharm. Sci. 53 (9) 1040-45 (1965)].

Das folgende Beispiel soll die Erfindung verdeutlichen.

### Beispiel: Herstellung von Mikrokristallen:

250 mg 4-Hydroxy-4-androsten-3,17-dion werden in 7,5 ml Aceton gelöst. Diese Lösung wird in einem Ultraschall-Desintegrator mit 75 ml Wasser vermischt. Die ausgefallenen Mikrokristalle des 4-Hydroxy-4-androsten-3,17-dions werden auf einem Filter durch Filtration gesammelt und zweimal mit je 10 ml Wasser nachgewaschen. Die gewaschenen Mikrokristalle werden bei 40°C und einem Druck von >100 mbar getrocknet.

## Patentansprüche

1. 4-Hydroxy- oder 4-Acyloxy-4-androsten-3,17-dione der allgemeinen Formel I 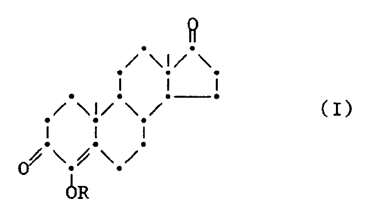 worin R entweder ein Wasserstoffatom oder eine 2 bis 12 Kohlenstoffatome enthaltende gesättigte aliphatische oder aromatische Acylgruppe bedeutet, gekennzeichnet dadurch, dass die Verbindung der allgemeinen Formel I in mikronisierter Form in einer Korngrösse der Grössenordnung von 2-20 µm vorliegt.

2. 4-Hydroxy- oder 4-C₂-C₁₂-Acyloxy-4-androsten- 3,17-dion der allgemeinen Formel I gemäss Anspruch 1, gekennzeichnet dadurch, dass die Verbindung der allgemeinen Formel I in mikronisierter Form in eine Korngrösse von 3-6 µm vorliegt.

3. Verfahren zur Herstellung einer Verbindung der allgemeinen Formel I in mikronisierter Form gemäss Anspruch 1 in einer Grössenordnung der Korngrösse von 2-20 µm, dadurch gekennzeichnet, dass man die Verbindung mittels eines Ultraschalldesintegrators mikronisiert.

## Claims

1. A 4-Hydroxy- or 4-acyloxy-4-androstene-3,17-dione of the general formula I 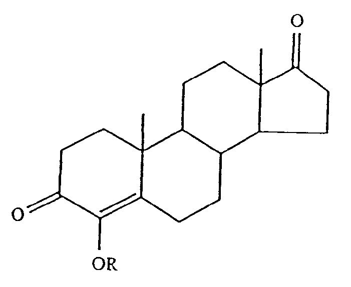 in which R represents either a hydrogen atom or a saturated aliphatic or aromatic acyl group of 2 to 12 carbon atoms, the compound of the general formula I in micronised form having a particle size of the order of magnitude of 2-20 µm.

2. A 4-hydroxy- or 4-C₂-C₁₂acyloxy-4-androstene-3,17-dione of the general formula I according to claim 1, the compound of the general formula I in micronised form having a particle size of 3-6 µm.

3. A method of preparing a compound of the general formula I in micronised form according to claim 1 in a particle size of the order of magnitude of 2-20 µm, which comprises micronising the compound with an ultrasonic disintegrator.

## Revendications

1. 4-hydroxy- ou 4-acyloxy-4-androstène-3,17-diones de formule générale I 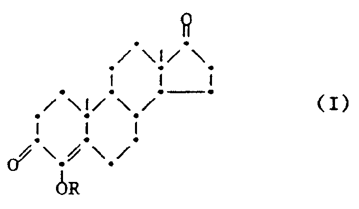 dans laquelle R représente un atome d'hydrogène ou un groupe acyle aliphatique saturé ou aromatique contenant 2 à 12 atomes de carbone, caractérisées en ce que le composé de formule générale I est à l'état micronisé à une dimension de grain d'un ordre de grandeur de 2 à 20 µm.

2. 4-hydroxy- ou 4-(alcoxy en C 2-C 12)-4-androstène-3,17-diones de formule générale I de la revendication 1, caractérisées en ce que le composé de formule générale I est à l'état micronisé à une dimension de grain de 3 à 6 µm.

3. Procédé de préparation d'un composé de formule générale I à l'état micronisé selon la revendication 1, à une dimension de grain d'un ordre de grandeur de 2 à 20 µm, caractérisé en ce que l'on soumet le composé à micronisation à l'aide d'un désintégrateur à ultrasons.
